# EUROPÄISCHE PATENTSCHRIFT

(11) **EP 1 440 677 B1**
(45) Veröffentlichungstag und Bekanntmachung des Hinweises auf die Patenterteilung: **25.06.2008**
(21) Anmeldenummer: 04450011.4
(22) Anmeldetag: 19.01.2004
(51) Int. Cl.: A61H 23/02, A61N 7/00

(54) **Schwingungsgenerator für akustischen Wellen mit Resonator und achsensymmetrischem Gehäuse**
Oscillator for acustic waves with resonator and axis-symmetric housing
Oscillateur des ondes acoustiques avec resonateur et boîtier axe-symmetrique

(30) Priorität: 22.01.2003 AT 822003
(43) Veröffentlichungstag der Anmeldung: 28.07.2004
(73) Patentinhaber: Luttermann, Joachim, 8082 Kirchbach (AT)
(72) Erfinder: Luttermann, Joachim, 8082 Kirchbach (AT)
(74) Vertreter: Gibler, Ferdinand

(56) Entgegenhaltungen:
- EP-A- 1 060 728
- DE-A- 3 324 575
- DE-C- 968 501
- RU-C- 2 188 618
- US-A- 4 909 240
- US-A- 6 131 256

## Beschreibung

Die Erfindung betrifft eine Vorrichtung zur Harmonisierung von natürlichen Schwingungen des menschlichen Körpers, mit einem Schwingungsgenerator, wobei der Schwingungsgenerator einen Schwingungsgenerator zur Erzeugung akustischer Wellen im nicht hörbaren Bereich und einen mit diesem gekoppelten akustischen Schallresonator umfasst.

Das vibrierende Erdkraftfeld eines Reizstreifens, einer Reizzone oder geopathischer Emanationen entsteht durch eine Erdkraftfeldstörung, die durch eine unterirdische Wasserader, eine tektonische Verwerfung im Untergrund, magnetische Erdfeldgitter, eine elektrische Kabelleitung, Mikrowellenstrahlung oder ähnliche vibrierende Strahlungsfelder verursacht werden. Reizstreifen oder geopathische Emanationen, wie sie über sogenannten Erdkraftfeldstörungen festgestellt werden können, wirken sich häufig ungünstig auf das Wohlbefinden von Mensch und Tier aus. Sie werden durch physikalische Vorgänge verursacht, die sich in der Erdrinde abspielen.

Die meisten Menschen sind durch die heutige Lebens- und Arbeitsweise gezwungen, sich großteils in geschlossenen Räumen aufzuhalten, die von elektromagnetischer Strahlung, die z.B. von Mobilfunk-Sendeanlagen herrühren, durchsetzt sind. Wenn nun Wohn- und Arbeitsräume von Reizzonen unterzogen sind, bilden sich auch sonstige Stör- und Schadwellen, deren Wirkung jene der Wellen technischen Ursprungs, wie z.B. Funkstrahlung, verstärkt. So ist das elektrische Potential oberhalb dieser Störwellen gestört, auch ist der pH-Wert der Luft in diesen Räumen gegen die Norm hin verschoben.

Der Organismus der Lebewesen wird durch längere oder dauernde Einwirkung solcher negativen Emanationszonen in seiner Abwehrkraft je nach Körperkonstitution mehr oder weniger geschwächt und kann dadurch pathogenen Einwirkungen nicht zur Genüge Widerstand leisten. Daher ermüden die meisten Menschen auf geopathischen Reizzonen und sonstigen Stör- und Schadwellen auch technischer Art sehr bald.

Ferner ist aus der US-A-4 909 240 ein Ultraschallkopf bekannt geworden mit einem Gehäuse mit einer Gehäuseöffnung, in der ein Ultraschallresonator auswechselbar einsetzbar ist, wobei der Ultraschallresonator in eine Öffnung eines proximalen Gehäuses eingesetzt ist, über das ein distales Gehäuse wenigstens teilweise darüberschiebbar ist.

Die EP 1 060 728 A zeigt eine Einrichtung zum Fettabbau eines lebenden Körpers mit Ultraschall, wobei u.a. ein Frequenzbereich von 15 bis 140 kHz vorgeschlagen wird.

Die US 6 131 256 A zeigt einen temperaturkompensierten Resonator und eine Methode zur Herstellung desselben, wobei der Resonator ein Substrat mit einem Hohlraum und einer Resonatorschicht umfasst. Ein Bindemittel koppelt das Substrat und die Resonatorschicht, wobei die Resonatorschicht auf dem Hohlraum angebracht ist. Ein elektrischer Leiter ist auf der Resonatorschicht angebracht, der mit elektrischem Strom beheizbar ist, um die Resonatorschicht zu beheizen.

Bisherige Versuche zur Ausschaltung des störenden Einflusses dieser Reizzonen beschränkten sich auf die Aussendung von elektromagnetischen Harmonisierungswellen mit sehr geringer Intensität mit relativ großen Schwankungen in der Frequenz, wodurch aber eine Besserung nur über einen relativ kurzen Zeitraum erzielbar war.

Aufgabe der Erfindung ist es daher, eine Vorrichtung der eingangs genannten Art anzugeben, mit der eine dauerhafte, positive Beeinflussung der durch die Erdkraftfeldstörungen bewirkten Reizzonen bewirkt werden kann, wobei die zu diesem Zweck abgegebene Strahlung eine über einen langen Zeitraum stabile Frequenz aufweist.

Erfindungsgemäß wird dies dadurch erreicht, dass der Schwingungsgenerator zur Erzeugung akustischer Wellen und der Schallresonator von einem aus einem nach einer Seite offenen Gehäusemantel, einer Deckfläche und einem Gehäusedeckel gebildeten Gehäuse umschlossen sind, dass die Deckfläche aus einem Sechseck gebildet ist, durch dessen Mittelpunkt die Längsachse des Gehäuses verläuft, und dass der Gehäusemantel in Form einer Sechsecksäule an die Deckfläche anschließt und dass der akustische Schallresonator durch einen Hohlzylinder aus Glas gebildet ist, der an seinem offenen Längsende durch eine Edelmetallplatte, vorzugsweise aus Silber, verschlossen ist, welche Edelmetallplatte im Inneren des Gehäuses mit der Deckfläche des Gehäuses verbunden ist, wobei die Achse des Hohlzylinders mit der Längsachse des Gehäuses fluchtet. '

Durch die Einwirkung der erfindungsgemäßen Vorrichtung wird das Vibrationsfeld des stark vibrierenden Raumes wieder harmonisch abgestimmt. Die durch Wechselwirkung des erzeugten akustischen Wellenfeldes mit der Erdkraftfeldstörung erreichte Interferenz dämpft die auf den Menschen wirkenden Störwellen so stark, daß die zellularen Vibrationen des menschlichen aber auch des tierischen und pflanzlichen Organismus ungestört bleiben. Mit Hilfe des Resonators kann die abgegebene Strahlung in ihrer Frequenz sehr genau abgestimmt werden und diese zeichnet sich durch hohe Langzeitstabilität aus. Es hat sich herausgestellt, daß ein Gehäuse mit einer sechsfachen Symmetrieachse die mit Abstand beste Harmonisierungswirkung für Lebewesen im Bereich der erzeugten akustischen Wellen ergibt. Die Wellenlängen der aus dem Erdkraftfeld eingekoppelten Raumstrahlen und sonstige Stör- und Schadwellen werden harmonisiert, so daß sie, ähnlich wie infrarote Strahlen nicht mehr schädigend auf Lebewesen einwirken können.

Die erfindungsgemäße Vorrichtung dient somit dem Ausgleich und der Unschädlichmachung von Störzonen und Emanationen, d.h. sie korrigiert und moduliert die pathogenen Emanationsvibrationen aus dem Untergrund sowie sonstige Stör- und Schadwellen, sodaß die natürlichen Schwingungen des menschlichen Organismus angeglichen werden. Sie verbessert die mikroklimatischen Verhältnisse im Aufstellungsbereich. Der Anwendungsbereich der erfindungsgemäßen Vorrichtung liegt in der Bereitstellung von gesunden Lebensräumen für Menschen, Tiere und Pflanzen. Häuser, Wohnungen, Büros, Industriekomplexe, Hotels, Krankenhäuser, Sanatorien, Operationssäle, Arzt-Praxen, sonstige öffentliche Gebäude, aber auch Ställe für Tiere, Gewächshäuser sowie auch Freilandbereiche sind auf diese Weise verbesserungsfahig.

Sowohl die Herstellung als auch die Wartung der erfindungsgemäßen Vorrichtung lassen sich weitgehend vereinfachen, wenn das Gehäuse aus einem nach einer Seite offenen Gehäusemantel, einer Deckfläche und einem Gehäusedeckel gebildet ist.

Eine herstellungstechnisch relativ einfache Bauform der erfindungsgemäßen Vorrichtung mit einer sechsfach-symmetrischen Achse besteht darin, daß die Deckfläche aus einem Sechseck gebildet ist, durch dessen Mittelpunkt die Längsachse des Gehäuses verläuft, und daß der Gehäusemantel in Form einer Sechsecksäule an die Deckfläche anschließt.

Da der Schallresonator bei der Erzeugung der Harmonisierungswellen eine zentrale Bedeutung hat, muß bei seiner Konstruktion besonders darauf geachtet werden, daß sein Aufbau besonders präzis auf die Frequenz der Harmonisierungswellen abgestimmt ist und die Verwendung von unedlen Metallen oder strahlungsdämpfenden Materialien vermieden wird. Es ist daher vorgesehen, daß der akustische Schallresonator durch einen Hohlzylinder aus Glas gebildet ist, der an seinem offenen Längsende durch eine Edelmetallplatte, vorzugsweise aus Silber, verschlossen ist, welche Edelmetallplatte im Inneren des Gehäuses mit der Deckfläche des Gehäuses verbunden ist, wobei die Achse des Hohlzylinders mit der Längsachse des Gehäuses fluchtet.

In weiterer Ausbildung der Erfindung kann der Schwingungsgenerator zur Erzeugung akustischer Wellen durch einen piezo-elektrischen Lautsprecher und einen mit diesem verbundenen Frequenzgenerator gebildet sein. Durch Verwendung des piezo-elektrischen Lautsprechers kann eine Einstrahlung beinahe ohne störende Resonanzerscheinungen des Lautsprechergehäuses der von diesem erzeugten akustischen Wellen in den Resonator erfolgen.

Die mit Abstand beste Harmonisierungswirkung kann mittels einer weiteren Ausführungsform der Erfindung erzielt werden, wenn der Frequenzgenerator ein Eingangssignal für den piezo-elektrischen Lautsprecher mit einer Frequenz von 128 000 Hz erzeugt.

Die Verstärkung des Energiefeldes der von der erfindungsgemäßen Vorrichtung erzeugten Hannonisierungsstrahlung kann dadurch erreicht werden, daß der Hohlzylinder kristallines Material und/oder Halbedelsteine und/oder Mineralien als Füllmaterial beinhaltet, welches als körniges Material vorliegt und vorzugsweise mit Lehm vermengt ist.

Ferner kann durch die Realisierung eines an sich bekannten Orgon-Aufbaus innerhalb des Resonators das Entstehen von Störresonanzen verhindert werden. Dies kann dadurch erzielt werden, daß das Füllmaterial von Wicklungslagen aus einer paramagnetischen Metall-Folie zumindest teilweise umgeben ist, und zwischen den Wicklungslagen aus Metall-Folie jeweils eine Isolationsfolie angeordnet ist, sodaß eine Schichtfolge aus Metall-Folie/Isolationsfolie/Metall-Folie das Füllmaterial umgibt.

Eine weitere Ausführungsform der Erfindung kann darin bestehen, daß das kristalline Material aus Bergkristallschrot, Rosenquarzschrot o.ä. gebildet ist. Durch die Kristalle und Halbedelsteine in Form von Schrot kann die Oberfläche der gesamten energetisch wirkenden Inhaltsstoffe des Hohlzylinders vergrößert werden, wodurch die Gesamtwirkung des Resonators verbessert wird.

Um die im Inneren des Füllmaterials sich ausbildenden Energiewirbel der Resonatorschwingungsenergie zuzuführen, kann in weiterer Ausbildung der Erfindung im Inneren des Hohlzylinders eine Drahtschleife aus Feinsilberdraht, insbesondere in Form einer Doppel-Helix, angeordnet sein, deren Enden mit der Edelmetallplatte verbunden sind.

Die spektrale Reinheit der Resonatorschwingung, welche die Grundlage der Ausbreitung der von der erfindungsgemäßen Vorrichtung erzeugten Harmonisierungswellen bildet, kann gemäß einem weiteren Ausführungsbeispiel der Erfindung dadurch verbessert werden, daß der Hohlzylinder aus Farbspektralglas, vorzugsweise Kobaltglas, gebildet ist.

In weiterer Fortbildung der Erfindung kann vorgesehen sein, daß der Schwingungsgenerator zur Erzeugung akustischer Wellen an dem von der Deckfläche abgewandten Ende des Hohlzylinders mit diesem akustisch gekoppelt ist. Auf diese Weise gelangt die eingekoppelte akustische Strahlung an der Stirnseite des Hohlzylinders in den Resonator, wodurch eine entlang der Längsachse des Hohlzylinders erfolgende Ausbreitung der sich ausbildenden stehenden Welle mit geringen seitlichen Reflexionen erfolgen kann.

Eine sehr stabile und zugleich die Ausbreitung der Harmonisierungswellen nicht behindernde Ausführungsform der erfindungsgemäßen Vorrichtung kann darin bestehen, daß das Gehäuse aus einer Keramik, aus Kunststoff o.ä. gebildet ist.

Nachfolgend wird die Erfindung anhand des in den Zeichnungen dargestellten Ausfilhrungsbeispiels eingehend erläutert. Es zeigt dabei
Fig.1 eine schematische Querschnittansicht einer Ausführungsform der erfindungsgemäßen Vorrichtung;
Fig.2 eine Draufsicht auf die in Fig. 1 gezeigte Vorrichtung;
Fig.3 eine Schrägrißdarstellung der Vorrichtung gemäß Fig.1 bei geöffnetem Deckel und
Fig.4 ein Schaltbild einer Ausführungsform des Frequenzgenerators.

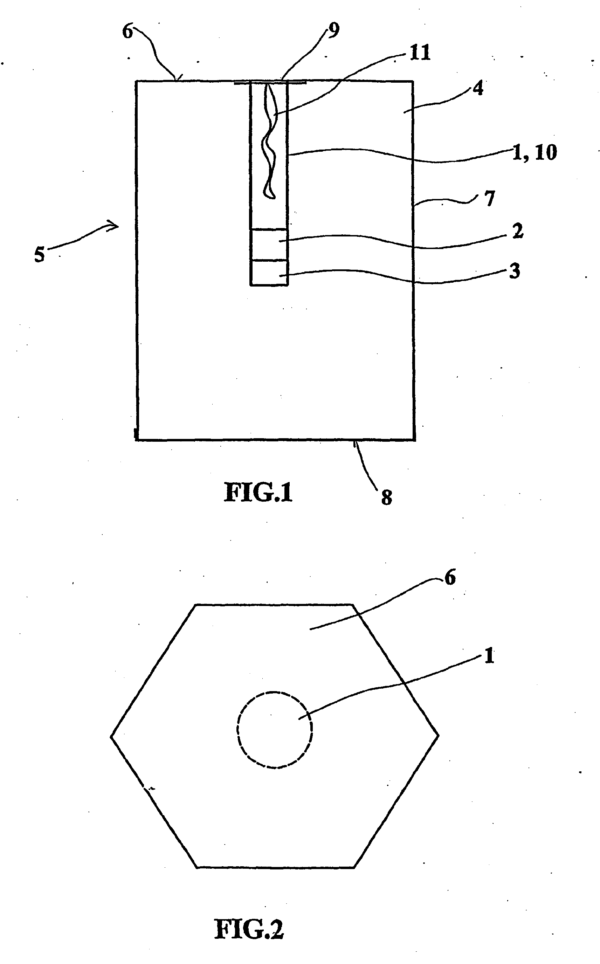

In Fig.1 ist ein Längsschnitt durch eine Vorrichtung zur Harmonisierung von natürlichen Schwingungen des menschlichen Körpers mit einer Schwingungs-Einheit 5 gezeigt, welche durch einen Schwingungsgenerator 2,3 zur Erzeugung akustischer Wellen im nicht hörbaren Bereich und einen mit diesem gekoppelten akustischen Schallresonator 1 gebildet ist. Der Schwingungsgenerator 2, 3 zur Erzeugung akustischer Wellen ist zusammen mit dem Schallresonator 1 von einem Gehäuse 4 umschlossen, das eine sechsfache Symmetrieachse aufweist. In Fig.2 ist dazu die obere Deckfläche 6 des Gehäuses 4 gezeigt, welche aus einem Sechseck gebildet ist.

Der Schwingungsgenerator zur Erzeugung akustischer Wellen ist durch einen piezo-elektrischen Lautsprecher 2 und einen mit diesem verbundenen Frequenzgenerator 3 gebildet, welcher ein Eingangssignal für den piezo-elektrischen Lautsprecher 2 mit einer Frequenz von 128 000 Hz erzeugt. Das vom piezo-elektrische Lautsprecher 2 erzeugte akustische Signal wird in den Schallresonator 1 eingekoppelt, in welchem eine Harmonisierungs-Schwingung ausgebildet wird, die durch das Gehäuse 4 in den umgebenden Raum abgestrahlt wird.

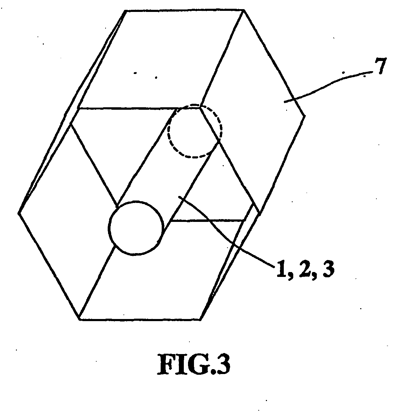

Gemäß dem in den Figuren gezeigten Ausführungsbeispiel ist das Gehäuse 4 aus einem nach einer Seite offenen Gehäusemantel 7, einer Deckfläche 6 und einem Gehäusedeckel 8 gebildet, der abnehmbar ist und über den ein Zugriff in das Innere des Gehäuses 4 möglich ist. Die einzelnen Teile des Gehäuses können z.B. aus einer Keramik, aus einem Kunststoff o.ä. gebildet sein. Fig.3 zeigt den Gehäusemantel 7 mit Deckfläche 6 ohne Gehäusedeckel 8.

Durch den Mittelpunkt der sechseckförmigen Deckfläche 6 verläuft die Längsachse des Gehäuses 4 und um diese herum der an die Deckfläche 6 anschließende Gehäusemantel 7 in Form einer Sechsecksäule.

Die Sechseck-Seitenlänge der sechseckförmigen Deckfläche 6 liegt vorzugsweise im Bereich von 12,5 cm bis 12,9 cm und die Höhe der Gehäusemantel-Sechsecksäule vorzugsweise 17,0 cm. Besonders bevorzugt ist eine Sechseck-Seitenlänge von 12,740 bis 12,756 cm. Diese Länge entspricht in etwa dem 10⁻⁸-fachen des Erddurchmessers und verbessert das Resonanzverhalten der erfindungsgemäßen Vorrichtung.

Als akustischer Schallresonator 1 ist ein Hohlzylinder 10 aus Glas ausgebildet, der an seinem offenen Längsende durch eine Edelmetallplatte 9, vorzugsweise aus Silber (999 Silber diamagnetisiert), verschlossen ist, die im Inneren des Gehäuses 4 mit der Deckfläche 6 des Gehäuses 4 verbunden ist, wobei die Achse des Hohlzylinders 10 mit der Längsachse des Gehäuses 4 fluchtet. Es kann aber der Resonator 1 im Rahmen der Erfindung auch eine andere geometrische Form aufweisen.

Im Inneren des Hohlzylinders 10 ist kristallines Material und/oder Halbedelsteine und/oder Mineralien als Füllmaterial beinhaltet, welches als körniges Material vorliegt und vorzugsweise mit Lehm vermengt ist. Das Füllmaterial kann aus allen existierenden Mineralien oder Mischungen davon gebildet sein, die z.B. in Form von Steinmehl mit dem Lehm vermengt sind. Der Resonator 1 kann aber auch frei von Füllmaterial ausgebildet sein.

Der Schwingungsgenerator 2, 3 zur Erzeugung akustischer Wellen ist an dem von der Deckfläche 6 abgewandten Ende des Hohlzylinders 10 mit diesem akustisch gekoppelt. Das Füllmaterial ist im Inneren des Hohlzylinders 10 von Wicklungslagen aus einer paramagnetischen Metall-Folie zumindest teilweise umgeben, wobei zwischen den Wicklungslagen aus Metall-Folie jeweils eine Isolationsfolie angeordnet ist, sodaß eine Schichtfolge aus Metall-Folie/Isolationsfolie/Metall-Folie das Füllmaterial umgibt. Die Schichtfolge kommt dabei zwischen der Wand des Hohlzylinders 10 und dem Füllmaterial zu liegen. Vorzugsweise ist die Schichtfolge durch dreizehn Wicklungslagen aus Metall-Folie und dazwischen angeordneter Isolationsfolie gebildet, sodaß ein 39-facher Orgonaufbau gewährleistet ist. Der dadurch gebildete Kondensator sammelt die aus der einstrahlenden elektromagnetischen Energie resultierende Ladung und gibt sie an das Füllmaterial ab.

Das kristalline Material kann aus Bergkristallschrot, Rosenquarzschrot o.ä. oder aus Mischungen davon gebildet sein, während die Halbedelsteine aus Amethystschrot, Aquamarinschrot o.ä. oder aus Mischungen davon bestehen können.

Im Inneren des Hohlzylinders 10 ist eine Drahtschleife 11 aus Feinsilberdraht mit einem Reinheitsgrad von 99,9% (999), insbesondere in Form einer Doppel-Helix, angeordnet, deren Enden mit der Edelmetallplatte 9 verbunden sind und die eine besonders hohe Energie-Bündelung innerhalb des Hohlzylinder-Resonators 10 bewirkt. Durch ungefähr zentrische Einbringung der Drahtschleife 11 in die zähe Füllmaterial-Lehmmischung wird diese durch die Hafteigenschaften des Lehms festgehalten. Zur weiteren Bündelung der im Hohlzylinder-Resonator 10 auftretenden Energie ist dieser aus Farbspektralglas, vorzugsweise Kobaltglas, gebildet, das vor allem im Raum außerhalb des Hohlzylinders 10 auftretende schädliche Einflüsse wie Elektrosmog, Erdstrahlen o.ä. neutralisiert.

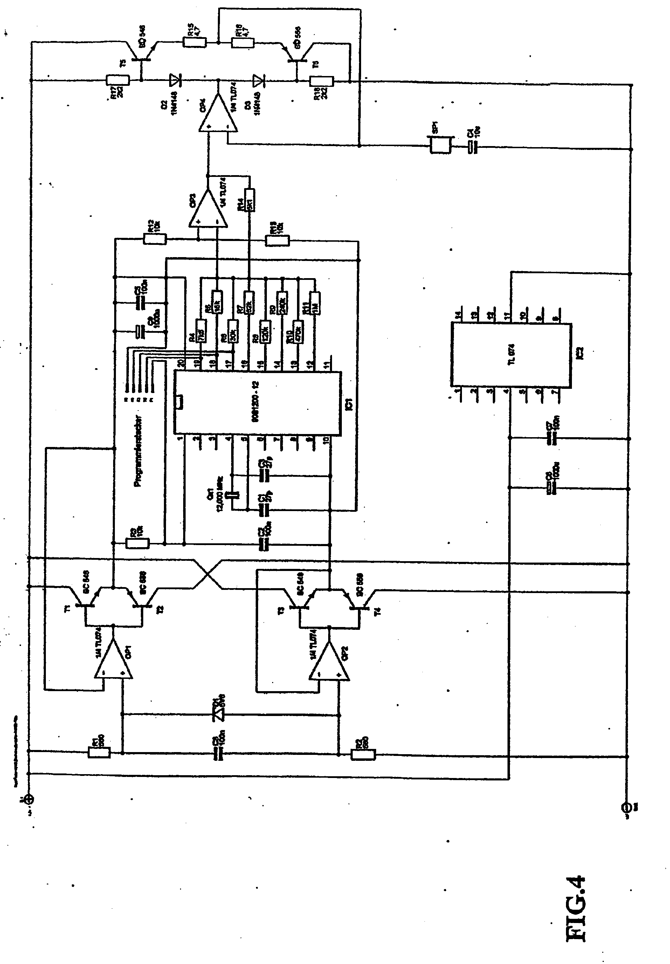

Der in der erfindungsgemäßen Vorrichtung beinhaltete Frequenzgenerator ist beispielhaft wie in Fig.4 ausgeführt. Es kann aber jede andere Form von Frequenzgenerator für die erfindungsgemäßen Zwecke verwendet werden, sofern dieser den piezo-elektrischen Lautsprecher mit einem Signal im nicht hörbaren Bereich beaufschlagen kann.

## Patentansprüche

1. Vorrichtung zur Harmonisierung von natürlichen Schwingungen des menschlichen Körpers, mit einer Schwingungs-Einheit, wobei die Schwingungs-Einheit (5) einen Schwingungsgenerator (2, 3) zur Erzeugung akustischer Wellen im nicht hörbaren Bereich und einen mit diesem gekoppelten akustischen Schallresonator (1) umfasst, **dadurch gekennzeichnet, dass** der Schwingungsgenerator (2, 3) zur Erzeugung akustischer Wellen und der Schallresonator (1) von einem aus einem nach einer Seite offenen Gehäusemantel (7), einer Deckfläche (6) und einem Gehäusedeckel (8) gebildeten Gehäuse (4) umschlossen sind, dass die Deckfläche (6) aus einem Sechseck gebildet ist, durch dessen Mittelpunkt die Längsachse des Gehäuses (4) verläuft, und dass der Gehäusemantel (7) in Form einer Sechsecksäule an die Deckfläche (6) anschließt und dass der akustische Schallresonator (1) durch einen Hohlzylinder (10) aus Glas gebildet ist, der an seinem offenen Längsende durch eine Edelmetallplatte (9), vorzugsweise aus Silber, verschlossen ist, welche Edelmetallplatte (9) im Inneren des Gehäuses (4) mit der Deckfläche (6) des Gehäuses (4) verbunden ist, wobei die Achse des Hohlzylinders (10) mit der Längsachse des Gehäuses (4) fluchtet.

2. Vorrichtung nach Anspruch 1, **dadurch gekennzeichnet, dass** der Sehwingungsgenerator zur Erzeugung akustischer Wellen durch einen piezo-elektrischen Lautsprecher (2) und einen mit diesem verbundenen Frequenzgenerator (3) gebildet ist.

3. Vorrichtung nach Anspruch 2, **dadurch gekennzeichnet, dass** der Frequenzgenerator ein Eingangssignal für den piezo-elektrischen Lautsprecher (2) mit einer Frequenz von 128.000 Hz erzeugt.

4. Vorrichtung nach einem der Ansprüche 1 bis 3, **dadurch gekennzeichnet, dass** der Hohlzylinder (10) kristallines Material und/oder Halbedelsteine und/oder Mineralien als Füllmaterial beinhaltet, welches als körniges Material vorliegt und vorzugsweise mit Lehm vermengt ist.

5. Vorrichtung nach einem der Ansprüche 1 bis 4, **dadurch gekennzeichnet, dass** das Füllmaterial von Wicklungslagen aus einer paramagnetischen Metall-Folie zumindest teilweise umgeben ist, und zwischen den Wicklungslagen aus Metall-Folie jeweils eine Isolationsfolie angeordnet ist, sodaß eine Schichtfolge aus Metall-Folie/Isolationsfolie/Metall-Folie das Füllmaterial umgibt.

6. Vorrichtung nach einem der Ansprüche 1 bis 5, **dadurch gekennzeichnet, dass** das kristalline Material aus Bergkristallschrot, oder Rosenquarzschrot gebildet ist.

7. Vorrichtung nach einem der Ansprüche 1 bis 6, **dadurch gekennzeichnet, dass** im Inneren des Hohlzylinders (10) eine Drahtschleife (11) aus Feinsilberdraht, insbesondere in Form einer Doppel-Helix, angeordnet ist, deren Enden mit der Edelmetallplatte (9) verbunden sind.

8. Vorrichtung nach einem der Ansprüche 1 bis 7, **dadurch gekennzeichnet, dass** der Hohlzylinder (10) aus Farbspektralglas, vorzugsweise Kobaltglas, gebildet ist.

9. Vorrichtung nach einem der Ansprüche 1 bis 8, **dadurch gekennzeichnet, dass** der Schwingungsgenerator (2, 3) zur Erzeugung akustischer Wellen an dem von der Deckfläche (6) abgewandten Ende des Hohlzylinders (10) mit diesem akustisch gekoppelt ist.

10. Vorrichtung nach einem der Ansprüche 1 bis 9, **dadurch gekennzeichnet, dass** das Gehäuse (4) aus einer Keramik oder aus Kunststoff gebildet ist.

## Claims

1. An apparatus for harmonizing natural oscillations of the human body, comprising an oscillation unit, with the oscillation unit (5) comprising an oscillator (2, 3) for generating acoustic waves in the non-audible range and an acoustic sound resonator (1) coupled with the same, **characterized in that** oscillator (2, 3) for generating acoustic waves and the sound resonator (1) are enclosed by a housing jacket (7) open towards one side, a cover surface (6) and a housing cover (8), that the cover surface (6) is formed by a hexagon, through the central point of which extends the longitudinal axis of the housing (4), and that the housing jacket (7) is adjacent to the cover surface (6) in the Form of a hexagonal column, and that the acoustic sound resonator (1) is formed by a hollow cylinder (10) made of glass which on its open longitudinal edge is sealed by a noble metal plate (9), preferably made of silver, which noble metal plate (9) is connected in the interior of the housing (4) with the cover surface (6) of the housing (4), with the axis of the hollow cylinder (10) being aligned with the longitudinal axis of the housing (4).

2. An apparatus according to claim 1, **characterized in that** the oscillator for generating acoustic waves is formed by a piezoelectric loudspeaker (2) and a frequency generator (3) connected with the same.

3. An apparatus according to claim 2, **characterized in that** the frequency generator generates an input signal for the piezoelectric loudspeaker (2) with a frequency of 128,000 Hz.

4. An apparatus according to one of the claims 1 to 3, **characterized in that** the hollow cylinder (10) contains crystalline material and/or semi-precious stones and/or minerals as filling material which is present as a granular material and is preferably mixed with clay.

5. An apparatus according to one of the claims 1 to 4, **characterized in that** the filling material is enclosed at least in part by winding layers of a paramagnetic metal foil, and an insulating foil each is arranged between the winding layers of metal foil, so that a layer sequence consisting of metal foil/insulating foil/metal foil encloses the filling material.

6. An apparatus according to one of the claims 1 to 5, **characterized in that** the crystalline material is made of quartz-crystal or rose-quartz grit.

7. An apparatus according to one of the claims 1 to 6, **characterized in that** a wire loop (11) made of fine-silver wire, especially in the form of a double helix, is arranged in the interior of the hollow cylinder (10), the ends of which are connected with the noble metal plate (9).

8. An apparatus according to one of the claims 1 to 7, **characterized in that** the hollow cylinder (10) is made of color spectral glass, preferably cobalt glass.

9. An apparatus according to one of the claims 1 to 8, **characterized in that** the oscillator (2, 3) for generating acoustic waves is acoustically coupled to the end of the hollow cylinder (10) averted from the cover surface (6).

10. An apparatus according to one of the claims 1 to 9, **characterized in that** the housing (4) is made of a ceramic or plastic material.

## Revendications

1. Dispositif pour l'harmonisation des vibrations naturelles du corps humain, avec une unité vibratoire, laquelle unité vibratoire (5) comprend un générateur de vibrations (2, 3) destiné à produire des ondes sonores dans la plage inaudible et avec un résonateur acoustique (1) couplé à celui-ci, **caractérisé en ce que** le générateur de vibrations (2, 3) destiné à produire des ondes sonores et le résonateur acoustique (1) sont enfermés dans un boîtier (4) formé d'une enveloppe de boîtier (7) ouverte sur un côté, d'une surface de couverture (6) et d'un couvercle de boîtier (8), **en ce que** la surface de couverture (6) est formée d'un hexagone au centre duquel passe l'axe longitudinal du boîtier (4), et **en ce que** l'enveloppe de boîtier (7) en forme de colonne hexagonale se raccorde à la surface de couverture (6) et **en ce que** le résonateur acoustique (1) est formé par un cylindre creux (10) en verre qui est fermé à son extrémité longitudinale ouverte par une plaque en métal noble (9), de préférence en argent, laquelle plaque de métal noble (9) est reliée à l'intérieur du boîtier (4) avec la surface de couverture (6) du boîtier (4), l'axe du cylindre creux (10) coïncidant avec l'axe longitudinal du boîtier (4).

2. Dispositif selon la revendication 1, **caractérisé en ce que** le générateur de vibrations destiné à produire des ondes sonores est formé par un haut-parleur piézoélectrique (2) et un générateur de fréquences (3) relié à celui-ci.

3. Dispositif selon la revendication 2, **caractérisé en ce que** le générateur de fréquences produit un signal d'entrée pour le haut-parleur piézoélectrique (2) à une fréquence de 128 000 Hz.

4. Dispositif selon l'une des revendications 1 à 3, **caractérisé en ce que** le cylindre creux (10) contient une matière cristalline et/ou des pierres semi-précieuses et/ou des minéraux servant de matériau de remplissage, qui se présentent sous forme de matériau granuleux et qui est de préférence mélangé à de l'argile.

5. Dispositif selon l'une des revendications 1 à 4, **caractérisé en ce que** le matériau de remplissage est entouré au moins partiellement par des couches enroulées d'une feuille métallique paramagnétique et il est prévu entre chaque enroulement de la feuille magnétique une feuille isolante, de sorte qu'une succession de couches de feuille métallique/feuille isolante/feuille métallique entoure le matériau de remplissage.

6. Dispositif selon l'une des revendications 1 à 5, **caractérisé en ce que** le matériau cristallin est formé de grenaille de cristal de roche ou de grenaille de quartz rose.

7. Dispositif selon l'une des revendications 1 à 6, **caractérisé en ce qu'**il est prévu à l'intérieur du cylindre creux (10) une boucle de fil (11) en argent fin, ayant en particulier la forme d'une double hélice, dont les extrémités sont reliées à la plaque en métal noble (9).

8. Dispositif selon l'une des revendications 1 à 7, **caractérisé en ce que** le cylindre creux (10) est formé de verre à spectre coloré, en particulier de verre au cobalt.

9. Dispositif selon l'une des revendications 1 à 8, **caractérisé en ce que** le générateur de vibrations (2, 3) destiné à produire des ondes sonores est couplé au cylindre creux (10) à l'extrémité de celui-ci opposée à la surface de couverture (6).

10. Dispositif selon l'une des revendications 1 à 9, **caractérisé en ce que** le boîtier (4) est fait d'une céramique ou d'une matière plastique.
